# EUROPEAN PATENT SPECIFICATION

(11) **EP 1 814 620 B1**
(45) Date of publication and mention of the grant of the patent: **06.07.2016**
(21) Application number: 05810634.5
(22) Date of filing: 21.11.2005
(51) Int. Cl.: A61M 16/04, A61M 25/10

(54) **SECRETION CLEARING VENTILATION CATHETER AND AIRWAY MANAGEMENT SYSTEM**
SEKRETENTFERNENDER BEATMUNGSKATHETER UND ATEMWEGS-MANAGEMENT-SYSTEM
CATHETER DE VENTILATION EVACUANT LES SECRETIONS ET SYSTEME DE GESTION DES VOIES AERIENNES

(30) Priority: 19.11.2004 US 629074 P
(43) Date of publication of application: 08.08.2007
(73) Proprietor: VERATHON MEDICAL (CANADA) ULC, Burnaby BC V5G 1B2 (CA)
(72) Inventor: PACEY, John, A., Vancouver, British Columbia V6J 4H2 (CA)
(74) Representative: Kalkoff & Partner
(86) International application number: PCT/CA2005/001783
(87) International publication number: WO 2006/053446

(56) References cited:
- EP-A2- 0 665 029
- EP-A2- 0 982 045
- WO-A1-03/055553
- WO-A2-02/089885
- CA-A1- 2 174 778
- DE-A1- 2 535 191
- US-A- 3 297 027
- US-A- 4 036 210
- US-A- 4 300 550
- US-A- 4 840 173
- US-A- 5 957 134

## Description

### Reference to Related Applications

This application claims priority to U.S. Provisional Application No. 60/629,074, filed on November 19, 2004.

### Field of the Invention

This invention relates to ventilation catheters commonly known as endotracheal tubes, which are used for intra-tracheal ventilation and the like.

### Background

The traditional field of airway management includes a process of controlled ventilation that usually uses a mechanical ventilating machine to deliver a predetermined amount of inspiratory fluid, which is usually an air/oxygen gas mixture, with or without added water vapor, to the lungs of a patient on a predetermined cycle. Usually, the ventilating machine cycles between delivering relatively high-pressure inspiratory fluid via a delivery system to the patient's lungs for a short time, and then reducing the pressure in the delivery system for a short time so that used inspiratory fluid within the patient's lungs is expelled. The ventilating machine repeats this cycle of delivering new inspiratory fluid to and then expelling used inspiratory fluid from a patient's lungs, thereby ensuring proper oxygenation of a patient during times when they are unable to breath on their own.

More recently, airway management systems have evolved to permit oxygenation of a patient using oxygenated liquids and/or using a non-cyclic process involving a continuous flow of oxygenated liquids to a patent's lungs while simultaneously maintaining a continuous flow of used fluids from the patient's lungs. An example of these types of systems can be found in U.S. Pat. No. 5,706,830 to Parker.

A ventilation catheter, which is often referred to as an endotracheal tube, is commonly used by both traditional and these more recent airway enough to allow a sufficient flow of oxygenated fluid therethrough.

To date, efforts to improve the use and operation of endotracheal tubes have focused on solving two problems. First, efforts have focused on improving the security and pneumatic sealing of the endotracheal tube within the trachea. Second, efforts have focused on improving the ability of the endotracheal tube to pneumatically isolate individual lungs and/or bronchial chambers within a lung.

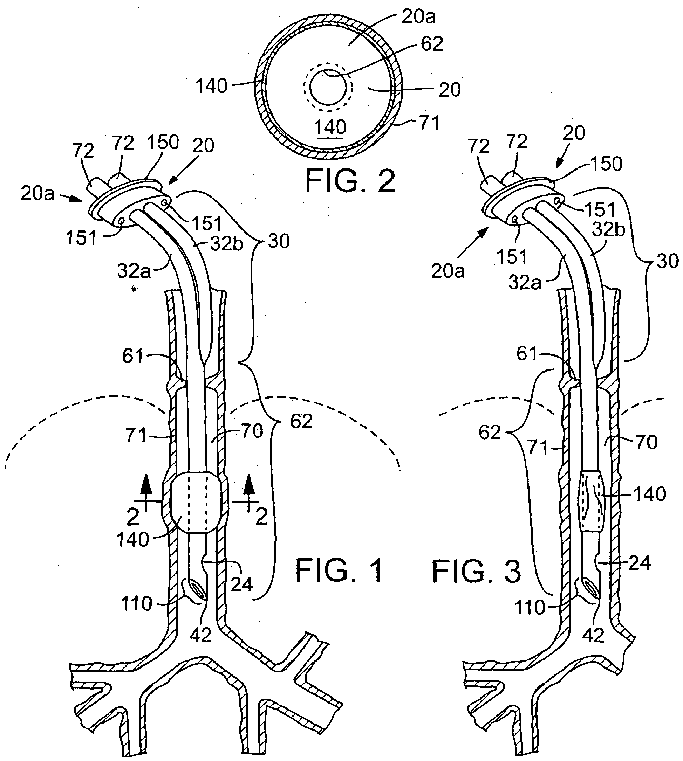

Regarding the first problem, one solution that addresses this issue has been to place an inflatable cuff around the endotracheal tube toward the distal and of the tube. The cuff is deflated during insertion of the tube, and inflated when the tube is properly positioned within the trachea, thereby holding the tube in place and creating a pneumatic seal. An example of these types of cuff structures can be found in FIGS. 1A and 1B of U.S. Pat. No. 6,443,156 to Niklason et al.

While the seal offered by these cuffs reduces the likelihood of a patent's airway being inadvertently contaminated with gastric and pharyngeal fluids, they also seal within a patient's lungs pulmonary secretions and fluids. A typical patient can produces about 200 cubic centimetres to 400 cubic centimetres of pulmonary secretions and fluids a day. The volume of these fluids and secretions tends to increase dramatically if a patent also has a pulmonary infection and/or certain types of cardiac disease.

The usual methods for addressing pulmonary secretion and fluid build-up arising during mechanical ventilation of a patient involve periodic suctioning of the patient's lungs and/or an increased antibiotic treatment to address ancillary infections that arise. Such periodic suctioning increases the risk of damaging a patient's pulmonary system and increases the risk of contaminating a patient's airway during each procedure.

Regarding the second problem, some inventors have attempted to isolate lungs and/or bronchial chambers by providing a plurality of individual lumens within the endotracheal tube. Each tube can have its own pneumatic cuff to allow isolation of particular lungs and/or bronchial tubes. However, each tube operates much like a single lumen tube, by providing both inspiratory fluid to the lung and removing used inspiratory fluid from the lung. These types of structures still allow pulmonary secretions and fluids to build-up in the lungs, and the traditional secretion removal and treatment methods must still be employed. Moreover, the cross section of the endotracheal tube can be rather large, thereby limiting the usefulness of the tube in small airways, such as on children and infants.

WO 02/089885 shows a respiration device with a double-lumen endotracheal tube, which is connected by two tubes to a respiration machine. The device further features sensors measuring gas pressure and flow in the two lumens and a evaluation unit for calculating the flow resistance based on the measured values.

WO 03/055553 discloses a system with an elongate member, like a respiration tube, that is to be inserted into a patient's trachea. At one end, a seal, e.g. a cuff, extends around the member. A wicking fluid pickup port is located beyond the seal and is coupled in fluid connection to a lumen that is arranged along the elongate member.

EP 0 982 045 discloses a bite protector for an airway, the protector having an elongate body and a longitudinally extending passage therethrough. The body is composed of two halves connected by a hinge. At one end, the protector features a flange and a reinforced wall portion adjacent to the flange to resist the bite action of a patient.

WO 95/13108 discloses a endotracheal tube for impeding air flow into a patient's lungs. The tube has, at a proximal end, an inflow lumen and an outflow lumen. At a distal end, the tube has single lumen. A one-way valve is located in each of the inflow and outflow lumen. The valves open when a certain amount of pressure is exceeded.

DE 2535191 discloses an endotracheal respiration system with a inspiration tube and an expiration tube. In between said tubes is disposed a third tube that serves to optionally divert gas from the inspiration tube directly into the expiration tube, e.g. when a patient breathes spontaneously. For this purpose, air pressure can be applied to the third tube, the end portion of which is located at a junction of the two tubes. The tubes are to be inserted up to the glottis of the patient.

US 5,957,134 discloses an anaesthesia delivery system comprising an endotracheal tube. The endotracheal tube has a portion with two lumens that are integrally formed with a wall in between, and a portion with a single lumen. The two lumens are used for supplying and expelling gases, respectively. The single-lumen portion preferably features an inflatable bulb element for securing the endotracheal tube in the trachea. Upstream of the endotracheal tube, the delivery system has a filter for exhaled air.

US 3,297,027 discloses a mouth-to-mouth respiration device, with an elongate, soft pliable tube that is to be introduced into the mouth of a person, and a second tube angularly disposed with respect to the first tube. The second tube is used by a person performing the respiration for blowing air into, while the first tube serves as an outlet for exhaled air. Since the diameter of the second tube is smaller than that of the first tube, mucus and vomiting that pass out with the outgoing air may pass through the first tube, while the second tube is unaffected. In one embodiment, the second tube extends inside the first tube up to its end.

### SUMMARY OF THE INVENTION

Accordingly, despite the benefits offered by known ventilation catheters, there is still a need for a compact endotracheal tube that can be easily inserted within a patient's trachea that allows for the easy removal of pneumatic secretions and liquids without the need for periodic auxiliary suctioning an the like. In addition to other benefits described herein, the present invention fulfils these needs.

The ventilation catheter according to the invention can be secured to a ventilation system that has a new inspiratory fluid path and an expiratory fluid path. The pulmonary secretion clearing ventilation catheter has a double lumen portion defining a first lumen and a second lumen separate from said first lumen, said double lumen portion sized to be received within a patient's mouth and extend down the patient's throat; and a single lumen portion formed from a joining together of the first lumen and the second lumen, the single lumen portion comprising an open distal end for allowing new inspiratory fluid from the ventilation system to flow into a patient and used inspiratory fluid from the patient to flow back to the ventilation system; said first lumen pneumatically connected to the new inspiratory fluid path; and said second lumen pneumatically connected to the expiratory fluid path, wherein said double lumen portion is sized to be positioned upstream of the patient's vocal chords while the diameter of the single lumen portion is adapted to allow the single lumen portion to extend past the patient's vocal chords into the patient's trachea. Thus, inspiratory fluid (air/oxygen mixtures, with or without added water vapor) is delivered to the distal end of the ventilation catheter through one of the two lumens and expired inspiratory fluid, pulmonary secretions, and pulmonary fluids are removed from the patent through the other lumen.

The used inspiratory fluid pathway preferably includes a secretion collection system for removing the pulmonary secretions and the like from the pathway thereby improving operation and safety of the system. In addition, by containing the used inspiratory fluid within the system, rather than releasing it to the environment, the release of potentially airborne infective material from a contagious patent, such as SARS and the like, can be minimized.

An improved cuff can also be used. The cuff encircles the distal end of the ventilation catheter to form a substantially pneumatic seal within the trachea. A small channel is formed along one side of the vent so as to allow a small leakage of air from the lungs of the patient during use to the ventilation catheter. This air leakage facilitates removal of secretions from within the patient's lungs without interfering with the ventilation catheter.

Other advantages and features of the present invention will become clear upon study of the following portion of this specification and drawings.

### BRIEF DESCRIPTION OF THE DRAWINGS

FIG. 1 is an isometric view of a first preferred ventilation catheter having a first inflatable cuff fin an inflated configuration in accordance with an embodiment of the present invention and showing a possible installation within a cut-away view of a patient's trachea.
FIG. 2 is a cross sectional view of the ventilation catheter of FIG. 1 taken along line 2-2 of FIG.1.
FIG. 3 is the ventilation catheter of FIG. 1 showing the first inflatable cuff deflated.
FIG. 4 is an isometric view of a second preferred ventilation catheter having a first inflatable cuff in an inflated configuration in accordance with an embodiment of the present invention.
FIG. 5 is a cross-sectional view of the ventilation catheter of FIG. 4 taken along line 5-5 of FIG. 4.
FIG. 6 is a schematic diagram of an airway management system with a ventilation catheter of FIG. 1 in accordance with an embodiment of the present invention.
FIG. 7 is a schematic diagram of an alternative preferred airway management system in accordance with an embodiment of the present invention showing a possible flow circuit of oxygenated fluid to a patient's lungs.
Fig. 8 is the schematic diagram of Fig. 7 showing a possible discharge circuit from the patient's lungs through the airway management system.
Fig. 9 is a schematic diagram of a third preferred ventilation catheter in accordance with an embodiment of the present invention.
Fig. 10 is the third preferred ventilation catheter of Fig. 8 showing a cuff in a possible deflated position.
Fig. 11 is an isometric view of a fourth preferred ventilation catheter showing a second preferred cuff operably attached thereto.
Fig. 12 is a cross-section view of the fourth preferred ventilation catheter of Fig. 11 taken along line 12-12 of Fig. 11.
Fig. 13 is an isometric view of a fifth preferred ventilation catheter in accordance with an embodiment of the present invention.

### Detailed Description of Preferred Embodiments

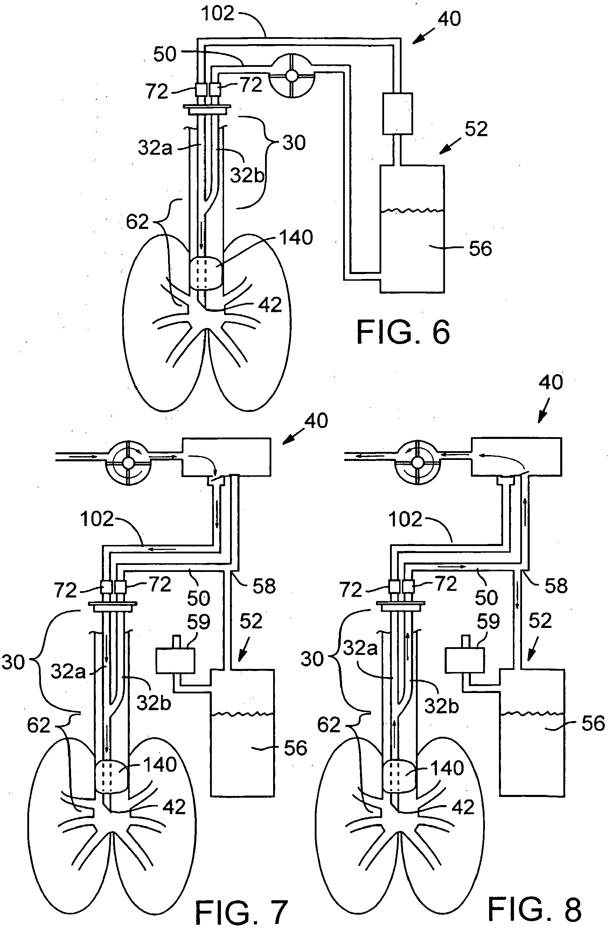

A pulmonary secretion clearing ventilation catheter 20 and related airway management system 22 are disclosed in Figs. 1-13. In general, the ventilation catheter 20, which is also referred to as an endotracheal tube, extends into the trachea 24 of a patient 26 to provide ventilation. The ventilation catheter 20 has a double lumen portion 30. Each lumen 32a, 32b of the double lumen portion 30 is operably secured to an airway management system 40 (Figs. 6-8) so that inspiratory fluid (air/oxygen mixtures, with or without added water vapor) is delivered to the distal end 42 of the ventilation catheter 20 through one of the two lumens 32a, 32b (here 32a is shown) and expired inspiratory fluid, pulmonary secretions, and pulmonary fluids are removed from the patent through the other lumen 32a, 32b (here 32b is shown).

Separating the incoming and outgoing inspiratory fluid flow through separate lumens 32a, 32b prevents the fresh incoming inspiratory fluid from becoming blocked or contaminated by inadvertent pulmonary secretions and fluids mixed with the used inspiratory fluid. The used inspiratory fluid pathway 50 (Figs. 6-8) preferably includes a secretion collection system 52 for removing the pulmonary secretions and the like from the pathway 50, thereby improving operation and safety of the system. In addition, by containing the used inspiratory fluid within the system, rather than releasing it to the environment, the release of potentially airborne infective material from a contagious patent, such as SARS and the like, can be minimized.

Several ventilation catheter embodiments having these basic features are disclosed in this application. In order to reduce undue repetition like elements between these embodiment have like element numbers.

Preferably and referring to Fig. 1, a first preferred ventilation catheter 20a is disclosed. In this embodiment, the double lumen portion 30 is positioned at the pre-pharyngeal/pharynx region of the patient upstream of their vocal chords 61. Each lumen 32a, 32b of the double lumen portion 30 pneumatically joins together to form a single lumen portion 62 that extends toward the distal end 42 of the ventilation catheter 20a. The single lumen portion 62 protrudes into the intratracheal region 70 of the patient. This allows a small diameter single lumen portion 62 to extend past the vocal chords into the trachea of the patent, while still allowing the secretion clearing benefits of the double lumen portion 30. The double lumen portion 30 positioned in the pre-pharyngeal level increases the efficiency of ventilation, reduces unnecessary dead space, eliminates secretions, and permits a smaller diameter intratracheal catheter to be used.

Preferably, each lumen 32a, 32b of the double lumen portion 30 has a proximal end 80 to which are connected conventional adapters 72 for detachable securing to mating connectors on a conventional ventilation system 40 (Fig. 6). One lumen 32a is connected through its connector to a delivery conduit 102 of a conventional ventilation system 100 (Fig. 6). The other lumen 32b is connected to the used inspiratory fluid pathway 50 of the ventilation system 100. The system 100 is controllable for delivering inspiratory fluid at pre-selected flow rates. The inspiratory fluid is thus delivered to the proximal end of lumen 32a at a pressure of about 20 mmHg for a selected period of time. After which, the system is vented in a way to drop the pressure in the system to about 6 mmHg during the expiratory phase of the ventilation cycle. This causes fluid, along with pulmonary secretions and pulmonary fluids to exit the patient through the second lumen 32b.

More preferably, the ventilation system 100 is configured to deliver inspiratory fluid through one lumen 32a of the double lumen portion 30 on inspiration while used inspiratory fluid and secretions are expelled from the patient through the other lumen 32b of the double lumen portion 30 during both inspiration and expiration phases of the ventilation system 40.

The tip 110 of the ventilation catheter 20a is preferably beveled and softened usefully to assist in the passage of the single lumen portion during the intubation of the trachea. The tip is preferably designed to resist the backward bending that might obstruct the airway.

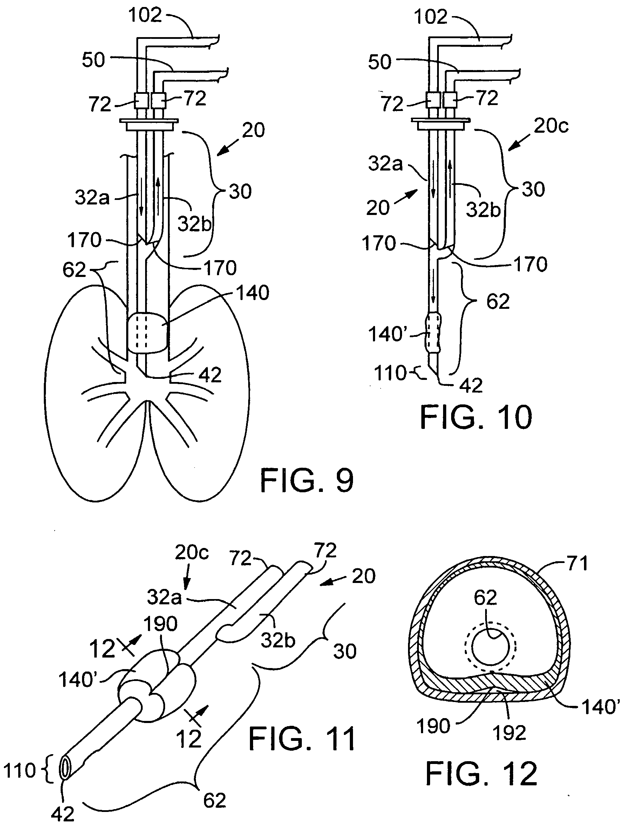

The double lumen portion and single lumen portion of the ventilation catheter are preferably made of a soft, clear medically approved elastomer. If desired and referring to Figs. 9, one-way check-valves 170, such as flapper valves, can be posited within one or both lumens of the double lumen portion to further prevent back-flow through these lumens.

Preferably, a low pressure, inflatable cuff 140 is positioned toward the distal end 42 of the ventilation catheter. The inflatable cuff 140 is preferably made of a thin film of substantially impermeable plastic or the like. The edges of the cuff are bonded to the outer surface of the single lumen portion 62. The cuff 140 is inflated with known means, such as those disclosed in Figs. 1A and 1B of U.S. Pat. No. 6,443,156, which involves extending a cuff inflation line within the ventilation catheter 20a from the cuff 140 to an auxiliary inflator.

The ventilation catheter 20a is inserted into a patient's trachea 24 when the cuff 140 is deflated as shown in Fig. 3. Then, the cuff 140 is inflated. Once inflated, the cuff 140 expands as shown in Figs. 1 and 2 to assume the substantially circular cross-sectional shape of the trachea, thereby pneumatically sealing the patient's lungs to the ventilation catheter 20a.

Preferably, a durable, bite-resistant, bite block 150 is secured to the double lumen portion of the ventilation catheter as shown in Fig. 1. Each lumen 32a, 32b of the double lumen portion 30 extends though the bite block 150. More preferably, the bite block 150 is slidably secured to the double lumen portion 30 so as to allow the position of the bite block 150 relative to a patient's teeth and lips to be adjusted as needed by sliding the bite block along the double lumen portion. More preferably, the bite block 150 includes one or more auxiliary holes therethrough or the like that are in parallel with the double lumen portion 30. These holes can be used as needed for endo-bronchial blocking catheters, fiberoptic endoscopes, and the like. Vent holes 151 can also be provided as needed.

Preferably, the ventilation system 40 (Fig. 6) is adapted as shown in Figs. 7 and 8 to include a secretion collection system 52 in the outflow circuit. The secretion collection system 52 preferably includes a secretion collection chamber 56 in pneumatic communication with the used inspiratory fluid pathway 50 at a diverter 58. As secretions in the used inspiratory fluid pathway 50 pass by the diverter 58, they are directed toward the collection chamber 56 either by gravity, or with the assistance of an auxiliary pump 59.

If desire, one or more ultraviolet light generating bulbs in the desired flow path(s) and the like to provide desired antibacterial activity as needed. Similarly, an appropriate antibacterial /anti-virus filter can be posted within the system to prevent exhaust gasses and the like from being released into the environment.

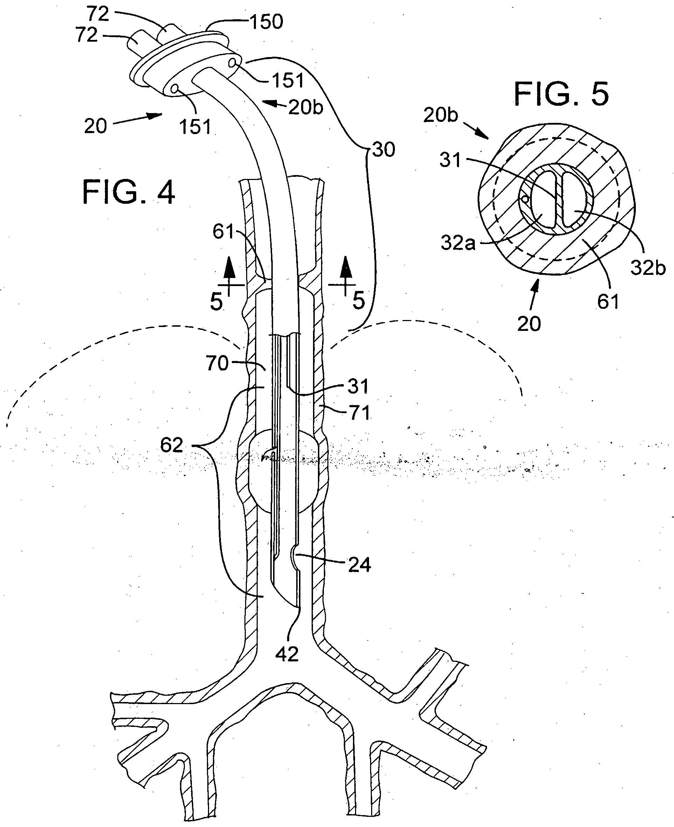

Referring to Figs. 4 and 5, a second preferred ventilation catheter 20b is shown. In this embodiment, the double lumen portion 30 is formed by a longitudinal wall 31 extending down the middle of a single lumen to define two lumens 32a, 32b. The wall 31 is removed toward the distal end 42 of the ventilation catheter 20b thereby combing the two lumens 32a, 32b into a single lumen portion 62. As with the first preferred ventilation catheter 20a (Figs. 1-3), oxygenate fluid in this embodiment 20b, is delivered through one lumen 32a to the single lumen portion 62, and used inspiratory fluid is removed from the patient through the second lumen 32b.

Referring to Figs. 10 and 11, a third preferred ventilation catheter 20c is shown. In this embodiment, the double lumen portion 30 is formed by a single lumen extending down the entire length of the ventilation catheter 20c, thereby defining a substantially straight channel through which to insert auxiliary devices, such as fiberoptic endoscopes, and the like. The second lumen 32b of the double lumen portion 30 intersects the single lumen substantially at a right angle as shown thereby defining the double lumen portion 30 of the ventilation catheter 20c.

As best shown in Figs. 11 and 12, an alternative preferred lower pressure, inflatable cuff 140' is positioned toward the distal end 42 of the ventilation catheter 20c. The alternative preferred inflatable cuff 140' is preferably made of a thin film of substantially impermeable plastic or the like. The edges of the cuff are bonded to the outer surface of the single lumen portion 62, and a recessed channel 190 is provided on one side of the inflated cuff 140'.

The cuff 140' is inflated with known means, such as those disclosed in Figs. 1A and 1B of U.S. Pat. No. 6,443,156, which involves extending a cuff inflation line within the ventilation catheter from the cuff to an auxiliary inflator.

The ventilation catheter 20c is inserted into a patient's trachea 24 without the cuff 140' inflated. Then, the cuff 140' is inflated. Once inflated, the cuff expands as shown in Figs. 11 and 12 to assume the substantially circular cross-sectional shape of the trachea 24, thereby substantially pneumatically sealing the patient's lungs to the ventilation catheter. The recessed channel 190 allows a small pneumatic opening 192 between the cuff 140' and the trachea wall 71, thereby allowing a limited pneumatic leak from the patient's lungs to the environment. Preferably, the channel 190 is sized so as to allow about 10% of the inspiratory fluid delivered through the ventilation catheter 20c to exit through the pneumatic opening 192.

This continuous leaking facilitates secretion clearing of the lungs. Pulmonary secretions and the like travel up the trachea through the pneumatic opening 192 in the cuff 141' to the patient's hypopharynx, where they can be easily suctioned way without disruption the ventilation catheter.

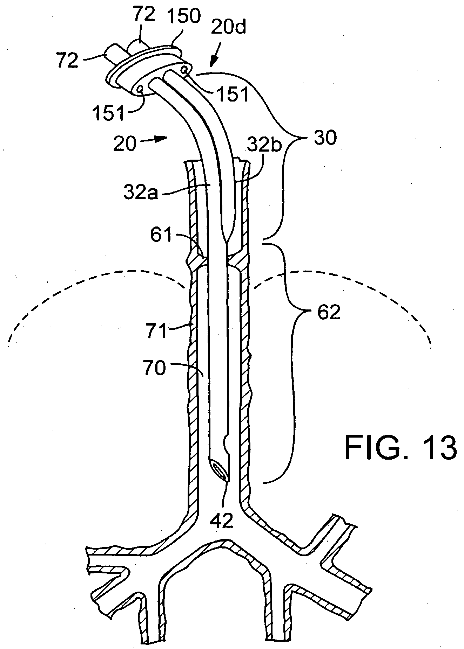

Referring to Fig. 13, a fourth ventilation catheter 20d is disclosed. This catheter is substantially similar to the first disclosed embodiment of Fig. 1, but does not have an inflatable cuff 140 (Fig. 1) operably secured thereto. This cuffless design is particularly useful when working in small tracheas, such as those found in infants and small children.

While the present invention has been described in terms of preferred embodiments, it will be appreciated by one of ordinary skill that the spirit and scope of the invention is not limited to those embodiments. For example, the alternative preferred cuff 140' (Figs. 10 & 11) and/or check valves 170 (Fig. 9) could be installed on any disclosed embodiment of the ventilation catheters 20a-d. Accordingly, the scope of the present invention extends to the various modifications and equivalents as defined in the appended claims.

## Claims

1. A ventilation catheter (20) for operably securing to a ventilation system (40) that has a new inspiratory fluid path (102) and an expiratory fluid path (50), said catheter (20) having:
- a double lumen portion (30) defining a first lumen (32a) and a second lumen (32b) separate from said first lumen (32a), said double lumen portion (30) sized to be received within a patient's mouth and extend down the patient's throat; and
- a single lumen portion (62) formed from a joining together of the first lumen (32a) and the second lumen (32b), the single lumen portion (62) comprising an open distal end (42) for allowing new inspiratory fluid from the ventilation system (40) to flow into a patient and used inspiratory fluid from the patient to flow back to the ventilation system (40);
said first lumen (32a) pneumatically connected to the new inspiratory fluid path (102); and said second lumen (32b) pneumatically connected to said expiratory fluid path (50),
**characterised in that**
the first lumen (32a) and the second lumen (32b) are disposed in two separate tubes and said double lumen portion (30) is sized to be positioned upstream of the patient's vocal chords (61), while the diameter of the single lumen portion (62) is adapted to allow the single lumen portion (62) to extend past the patient's vocal chords (61) into the patient's trachea (70).

2. The ventilation catheter of claim 1, wherein said second lumen (32b) intersects substantially perpendicular with said first lumen (32a), thereby defining a substantially straight path from said first lumen (32a) through said single lumen portion (62).

3. The ventilation catheter of claim 1, further including an inflatable cuff (140,140') operably secured to the single lumen portion (62).

4. The ventilation catheter of claim 3, wherein said inflatable cuff (140') has an elongate channel (190) along an outer surface, thereby defining an airway leaking channel (192) when the cuff is inflated in the trachea (70) of a patient.

5. The ventilation catheter of claim 4, wherein the airway leaking channel (192) is sized to allow about 10% of the inspiratory fluid flow provided by the ventilation system (40) to leak therethrough.

6. The ventilation catheter of claim 1, further including a bite protector (150) slidably secured to the double lumen portion (30).

7. The ventilation catheter of claim 6, wherein said bite protector (150) includes at least one channel (151) therethrough to permit access to the new inspiratory fluid path (102) within the ventilation catheter (20).

8. The ventilation catheter of claim 1, wherein said new inspiratory fluid is a gas.

9. The ventilation catheter of claim 1, wherein said expiratory fluid path (50) includes a secretion collector (52) for collecting pulmonary secretions and pulmonary fluids collected from the patient through the expiratory fluid path (50).

10. The ventilation catheter of claim 9, wherein said secretion collector (52) includes a secretion chamber (56) for collecting secretions therein.

11. The ventilation catheter of claim 1, further including a check-valve operably secured to at least one said first lumen and said second lumen to prevent inadvertent pneumatic back flow.

## Patentansprüche

1. Beatmungskatheter (20) zum betriebsfähigen Befestigen an einem Beatmungssystem (40), das einen neuen inspiratorischen Fluidpfad (102) und einen expiratorischen Fluidpfad (50) aufweist, wobei der Katheter (20) Folgendes aufweist:
- einen Doppellumenabschnitt (30), der ein erstes Lumen (32a) und ein zweites Lumen (32b) definiert, das von dem ersten Lumen (32a) getrennt ist, wobei der Doppellumenabschnitt (30) bemessen ist, um innerhalb des Mundes eines Patienten aufgenommen zu werden und sich den Hals des Patienten hinab zu erstrecken; und
- einen Einzellumenabschnitt (62), der durch ein Zusammenverbinden des ersten Lumens (32a) und des zweiten Lumens (32b) gebildet ist, wobei der Einzellumenabschnitt (62) ein offenes distales Ende (42) umfasst, um das Strömen eines neuen inspiratorischen Fluides von dem Beatmungssystem (40) in einen Patienten und das Strömen von gebrauchtem inspiratorischem Fluid von dem Patienten zurück zu dem Beatmungssystem (40) zu ermöglichen;
wobei das erste Lumen (32a) pneumatisch mit dem neuen inspiratorischen Fluidpfad (102) verbunden ist;
und das zweite Lumen (32b) pneumatisch mit dem expiratorischen Fluidpfad (50) verbunden ist,
**dadurch gekennzeichnet, dass**:
das erste Lumen (32a) und das zweite Lumen (32b) in zwei getrennten Röhren angeordnet sind und der Doppellumenabschnitt (30) bemessen ist, um den Stimmbändern (61) des Patienten vorgelagert positioniert zu sein, während der Durchmesser des Einzellumenabschnitts (62) angepasst ist, um es dem Einzellumenabschnitt (62) zu ermöglichen, sich an den Stimmbändern (61) des Patienten vorbei in die Luftröhre (70) des Patienten zu erstrecken.

2. Beatmungskatheter nach Anspruch 1, wobei das zweite Lumen (32b) sich im Wesentlichen senkrecht mit dem ersten Lumen (32a) kreuzt, wodurch ein im Wesentlichen gerader Pfad von dem ersten Lumen (32a) durch den Einzellumenabschnitt (62) definiert wird.

3. Beatmungskatheter nach Anspruch 1, der ferner eine aufblasbare Manschette (140, 140') umfasst, die betriebsfähig am Einzellumenabschnitt (62) befestigt ist.

4. Beatmungskatheter nach Anspruch 3, wobei die aufblasbare Manschette (140') einen länglichen Kanal (190) entlang einer Außenfläche aufweist, wodurch ein undichter Atemwegskanal (192) definiert wird, wenn die Manschette in der Luftröhre (70) eines Patienten aufgeblasen wird.

5. Beatmungskatheter nach Anspruch 4, wobei der undichte Atemwegskanal (192) bemessen ist, um es etwa 10% des inspiratorischen Fluidstoms, der durch das Beatmungssystem (40) bereitgestellt wird, zu erlauben, dort hindurch auszutreten.

6. Beatmungskatheter nach Anspruch 1, das ferner einen Beißschutz (150) umfasst, der verschiebbar an dem Doppellumenabschnitt (30) befestigt ist.

7. Beatmungskatheter nach Anspruch 6, wobei der Beißschutz (150) mindestens einen Kanal (151) durch ihn hindurch umfasst, um den Zugang zu dem neuen inspiratorischen Fluidpfad (102) innerhalb des Beatmungskatheters (20) zu ermöglichen.

8. Beatmungskatheter nach Anspruch 1, wobei das neue inspiratorische Fluid ein Gas ist.

9. Beatmungskatheter nach Anspruch 1, wobei der expiratorische Fluidpfad (50) einen Sekretsammler (52) zum Sammeln von Lungensekreten und Lungenfluiden umfasst, die von dem Patienten durch den expiratorischen Fluidpfad (50) gesammelt werden.

10. Beatmungskatheter nach Anspruch 9, wobei der Sekretsammler (52) eine Sekretkammer (56) zum Sammeln von Sekreten darin umfasst.

11. Beatmungskatheter nach Anspruch 1, der ferner einen Rückflussverhinderer umfasst, der betriebsfähig an mindestens einem von dem ersten Lumen und dem zweiten Lumen befestigt ist, um ein unbeabsichtigtes pneumatisches Rückströmen zu verhindern.

## Revendications

1. Cathéter de ventilation (20) destiné à être fixé fonctionnellement à un système de ventilation (40) qui comporte une voie de fluide d'inspiration neuf (102) et une voie de fluide d'expiration (50), ledit cathéter (20) comportant :
- une portion à double lumière (30) définissant une première lumière (32a) et une deuxième lumière (32b) séparée de ladite première lumière (32a), ladite portion à double lumière (30) étant dimensionnée pour être reçue dans la bouche d'un patient et s'étendant dans la gorge du patient ; et
- une portion à lumière unique (62) formée en joignant entre elles la première lumière (32a) et la deuxième lumière (32b), la portion à lumière unique (62) comprenant une extrémité distale ouverte (42) pour permettre à un fluide d'inspiration neuf provenant du système de ventilation (40) de s'écouler dans un patient et à un fluide d'inspiration usé provenant du patient de refluer vers le système de ventilation (40) ;
ladite première lumière (32a) étant connectée pneumatiquement à la voie de fluide d'inspiration neuf (102) ; et
ladite deuxième lumière (32b) étant connectée pneumatiquement à ladite voie de fluide d'expiration (50),
**caractérisé en ce que**
la première lumière (32a) et la deuxième lumière (32b) sont agencées dans deux tubes séparés et ladite portion à double lumière (30) est dimensionnée pour être positionnée en amont des cordes vocales (61) du patient, alors que le diamètre de la portion à lumière unique (62) est adapté pour permettre à la portion à lumière unique (62) de s'étendre au-delà des cordes vocales (61) du patient dans la trachée (70) du patient.

2. Cathéter de ventilation selon la revendication 1, dans lequel ladite deuxième lumière (32b) intersecte ladite première lumière (32a) de manière sensiblement perpendiculaire, définissant ainsi une voie sensiblement droite à partir de ladite première lumière (32a) à travers ladite portion à lumière unique (62).

3. Cathéter de ventilation selon la revendication 1, comprenant en outre un manchon gonflable (140, 140') fixé fonctionnellement à la portion à lumière unique (62).

4. Cathéter de ventilation selon la revendication 3, dans lequel ledit manchon gonflable (140') comporte un canal allongé (190) le long d'une surface externe, définissant ainsi un canal de fuite de voie aérienne (192) quand le manchon est gonflé dans la trachée (70) d'un patient.

5. Cathéter de ventilation selon la revendication 4, dans lequel le canal de fuite de voie aérienne (192) est dimensionné pour permettre la fuite à travers celui-ci d'environ 10 % du débit de fluide d'inspiration délivré par le système de ventilation (40).

6. Cathéter de ventilation selon la revendication 1, comportant en outre un protecteur de morsure (150) fixé de façon coulissante à la portion à double lumière (30).

7. Cathéter de ventilation selon la revendication 6, dans lequel ledit protecteur de morsure (150) comprend à travers celui-ci au moins un canal (151) pour permettre l'accès à la voie de fluide d'inspiration neuf (102) dans le cathéter de ventilation (20).

8. Cathéter de ventilation selon la revendication 1, dans lequel ledit fluide d'inspiration neuf est un gaz.

9. Cathéter de ventilation selon la revendication 1, dans lequel ladite voie de fluide d'expiration (50) comprend un collecteur de sécrétion (52) pour collecter des sécrétions pulmonaires et des fluides pulmonaires collectés auprès du patient à travers la voie de fluide d'expiration (50).

10. Cathéter de ventilation selon la revendication 9, dans lequel ledit collecteur de sécrétion (52) comprend une chambre de sécrétion (56) pour y collecter des sécrétions.

11. Cathéter de ventilation selon la revendication 1, comprenant en outre un clapet anti-retour fixé fonctionnellement à au moins une lumière parmi ladite première lumière et ladite deuxième lumière pour éviter un reflux pneumatique accidentel.
